# EUROPÄISCHE PATENTSCHRIFT

(11) **EP 0 298 438 B1**
(45) Veröffentlichungstag und Bekanntmachung des Hinweises auf die Patenterteilung: **18.11.1993**
(21) Anmeldenummer: 88110758.5
(22) Anmeldetag: 06.07.1988
(51) Int. Cl.: C07H 5/02, C12P 19/14, C12P 19/16

(54) **Disaccharidfluoride, Verfahren zur enzymatischen Herstellung mit alpha-glycosylfluoriden als Substraten**
Disaccharidyl fluorides and process for enzymatic synthesis using alpha-glycosyl fluorides as substrates
Fluorures disaccharidyles et procédé de synthèse enzymatique utilisant des florures alpha-glycosyles comme substrats

(30) Priorität: 10.07.1987 DE 3722812
(43) Veröffentlichungstag der Anmeldung: 11.01.1989
(73) Patentinhaber: HOECHST AKTIENGESELLSCHAFT, 65926 Frankfurt am Main (DE)
(72) Erfinder: Schlingmann, Merten, Prof. Dr., D-6240 Königstein/Taunus (DE); Keller, Reinhold, Dr., D-6232 Bad Soden am Taunus (DE); Wiesner, Matthias, Dr., D-6500 Mainz (DE); Treder, Wolfgang, Dr., D-4400 Münster (DE); Thiem, Joachim, Prof. Dr., D-4400 Münster (DE)

(56) Entgegenhaltungen:
- EP-A- 0 226 563
- Arch. Biochem. Biophys. 142, 382-393 (1971)
- THE JOURNAL OF BIOLOGICAL CHEMISTRY, Band 256, Nr. 12, 25. Juni 1981, Seiten 6017-6026; S. KITAHATA et al.: "Scope and mechanism of carbohydrase action"
- CARBOHYDRATE RESEARCH, Band 71, 1979, Seiten 287-298, Elsevier Scientific Publishing Co., Amsterdam, NL; G. OKADA et al.: "The predominantly nonhydrolytic action of alpha amylases on alpha-maltosyl fluoride"

## Beschreibung

Enzymatische Glycosylierungsmethoden finden in jüngster Zeit immer stärkere Beachtung, da sich auf diese Weise unter Vermeidung langer Synthesewege und aufwendiger Schutzgruppentechniken gezielt Oligosaccharide aufbauen lassen [G.M. WHITESIDES, C.-H. WONG Angew. Chem. 97, 617 (1985)]. Hierbei ist es oft möglich, aufgrund der bekannten biologischen Wirkung der Enzyme, die entstehenden Produkte hinsichtlich ihrer Verknüpfungsart und -stelle vorherzusagen. Besondere Bedeutung haben hier die Glycosyltransferasen [C.-H. WONG, S. HAYNIE, G.M. WHITESIDES J.Org. Chem. 47, 5416 (1982); J. THIEM, W. TREDER Angew. Chem. 98, 1100 (1986)] sowie die Transglycosylierungen bewirkenden Glycosylasen erlangt [(L. HEDBYS, P.O. LARSSON, K. MOSBACH, S. SVENSSON Biochem. Biophys. Res. Commun. 123, 8 (1984)]. Seit den Arbeiten von Barnett [J.E.G. BARNETT, W.T.S. JARVIS, K.A. MUNDAY Biochem. J. 103, 699 (1967) und 105, 699 (1967); J.E.G. BARNETT Biochem. J. 123, 607 (1971)] ist bekannt, daß Glycosylfluoride von den jeweils spezifischen Glycosylhydrolasen zu Fluorwasserstoff und den entsprechenden Kohlenhydraten gespalten werden. Seither sind eine Reihe von Enzymen gefunden worden, die Glycosylfluoride als Substrat akzeptieren.

So läßt sich mit Sucrose-Phoshorylase aus α-D-Glucosylfluorid in Gegenwart von Phosphat das Glucose-1-phosphat gewinnen [A.M. GOLD, M.P.OSBER Biochem. Biophys. Res. Commun. 42, 469 (1971)], ein glycogenartiges Polysaccharid aus α-D-Glucosylfluorid und Fructose durch Amylosucrase herstellen [G.OKADA, E.J. HEHRE Carbohydr. Res. 26, 240 (1973)], oder mit Cyclodextrin-α(1→4)-Glycosyltransferase α- und β-Cyclodextrin neben Maltooligomeren aufbauen (Deutsche Patentanmeldung P 36 26 213 7). Auch Disaccharid-Fluoride können als Substrate eingesetzt werden, wie es die Bildung von Maltooligomeren aus α-Maltosylfluorid gezeigt hat [S. KITAHATA, C.F. BREWER, D.S. GENGHOF, T. SAWAI, E.J. HEHRE J. Biol. Chem. 256, 6017 (1981)].

Aus Arch. Biochem. Biophys. 142, S. 382-393 (1971) ist die katalytische Aktivität von α-Amylasen, die zur Gruppe der α-Glycosidasen gehören, bekannt. Die Autoren beschreiben, daß α-Amylasen die Synthese von Maltose bzw. Maltosacchariden aus α-Glucopyranosylfluorid bzw. aus α-D-Glucopyranose katalysieren. Die Umsetzung wird jedoch nicht im präparativen Maßstab durchgeführt.

Die Umsetzung von α-Methylglycosiden oder α-Glycosylfluoriden mit Hilfe von Glucodextranase (Glycosidase) zu α-Methylisomaltose wird im J. of Biol. Chem., Vol. 256, No. 12, S. 6017-6026 (Kitahata et al.) beschrieben.

In EP-A-0 226 563 ist die enzymatische Synthese von Oligosaccharidverbindungen beschrieben. Als Enzyme werden α- und β-Glycosidasen eingesetzt.

Bei den beiden letztgenannten Umsetzungen entstehen Verbindungen, die am C-1 Kohlenstoff eine -OMe-Gruppe besitzen. Der Einsatz dieser Verbindungen für weitere Glykosilierungesreaktionen ist aufgrund der -OMe-Gruppe sehr schwierig.

Es wurde nun überraschend gefunden, daß die Inkubation von α-D-Glycosylfluorid mit α-Glucosidase oder α-Galactosidase in Reaktionslösungen mit 30 bis 60 Gew.-% Substrat neben der Spaltung zu dem entsprechenden Monosaccharid und Fluorwasserstoff sowie der Bildung von Oligomeren auch zu der Bildung von Disaccharidfluoriden führt.

Die Erfindung betrifft somit ein Verfahren zur Herstellung von Disaccharidfluoriden, das dadurch gekennzeichnet ist, daß α-D-Glycosylfluoride in Reaktionslösungen mit 30 bis 60 Gew.-% Substrat mit α-Glycosidase oder α-Galactosidase umgesetzt werden.

Im folgenden wird die Erfindung detailliert erläutert bzw. in den Patentansprüchen definiert.

Durch Inkubation mit α-Glycosidase oder α-Galactosidase erhält man in Reaktionslösungen mit 30 bis 60 Gew.-% Substrat der entsprechenden α-Glycosylfluoride in überraschender Weise Oligomere, die auch noch anomer gebundenes Fluor enthalten können. Die Glycosylfluoride sollten in der Reaktionslösung in Konzentrationen von ca. 30 bis 60 %, bevorzugt 40 bis 50 %, insbesondere 45 %, bezogen auf das Gewicht der Lösung, enthalten sein.

Das Enzym kann sowohl in freier Form als auch in immobilisierter Form eingesetzt werden. Wegen der besseren Raum/Zeit Ausbeute ist die immobilisierte Form bevorzugt. Zu diesem Zweck wird das Enzym im allgemeinen adsorptiv oder kovalent mit oder ohne Spacer an einen Träger gebunden. Geeignet sind organische Träger, wie beispielsweise Polyacrylnitril, oder anorganische Träger, wie z.B. Kieselgel. Besonders bevorzugt ist die kovalente Bindung des Enzyms an den Träger über einen Spacer, insbesondere Glutardialdehyd.

Die Umsetzung des α-Glycosylfluorids erfolgt in wäßriger Lösung oder in wäßrig/organischen Gemischen, wobei das Verhältnis von wäßriger Lösung zu organischem Lösungsmittel im Bereich 2:1 bis 1:2, bevorzugt bei 1:1 liegt. Geeignete Lösungsmittelgemische sind beispielsweise Wasser/(C₁-C₄) Alkanole, Wasser/Acetonitril oder Wasser/Aceton. Die Reaktion erfolgt im allgemeinen bei Temperaturen von 20 bis 60°C, insbesondere bei 30 bis 40°C, und bei einem pH-Wert von 5 bis 8, bevorzugt bei pH 6 bis 7. Wegen des freiwerdenden Fluorwasserstoffs ist es wichtig, den pH-Wert während der Reaktion zu kontrollieren und beispielsweise durch Zugabe von festem Natriumhydrogencarbonat oder Kationentauschern bei pH 6 bis 7 zu halten.

Die Reaktionszeit ist abhängig von der für die Reaktion gewählten Temperatur. Arbeitet man in dem bevorzugten Temperaturbereich, so ist eine Reaktionszeit von ca. 2 bis 4 Stunden zu erwarten.

Als Substrate werden bevorzugt α-D-Glucosylfluorid bzw. α-D-Galactosylfluorid eingesetzt. Bei der erfindungsgemäßen Umsetzung von α-D-Glucosylfluorid mit α-Glucosidase entsteht neben Glucose und Fluorwasserstoff sowie den 15 Oligomeren Isomaltose und Panose auch die bislang noch nicht beschriebene Verbindung Isomaltosylfluorid, wohl durch Übertragung eines Glucoserestes auf α-D-Glucosylfluorid unter Erhalt der Kohlenstoff-Fluor-Bindung. Als aktiviertes Disaccharid ist es von besonderem Interesse 20 für chemische und enzymatische Reaktionen und kann auf herkömmliche Weise nur schwierig hergestellt werden.

Die analoge Reaktion des α-D-Galactosylfluorids mit α-Galactosidase liefert das bisher nicht bekannte Galactobiosylfluorid neben anderen Oligomeren.

In den folgenden Beispielen wird die Erfindung weiter im Detail erläutert. Prozentangaben beziehen sich auf das Gewicht, wenn nicht anders angegeben.

### Beispiel 1

### Umsetzung von α-D-Glucopyranosylfluorid

### 1. Immobilisierung des Enzyms

α-Glucosidase (α-D-Glucosidglucohydrolase, E.C. 3.2.1.20) (57 U) werden in 10 ml eines 0,05 M Natriumacetatpuffers (pH 6,5) gelöst und bei 20°C 4 h mit 5 g eines mit Glutardialdehyd funktionalisierten Kieselgelträgers (Grace 332 250 A) geschüttelt [H.H. Weetall, Meth. Enzymol. 44, 134 (1976)]. Anschließend wird das Gel mit 100 ml bidest. Wasser, 200 ml M NaCl und wiederum 200 ml bidest. Wasser gewaschen. Das immobilisierte Enzym ist nun für die Reaktion direkt einsetzbar. Das Gel kann länger als vier Wochen ohne Aktivitätsverlust bei 4°C aufbewahrt werden.

### 2. Synthese der Oligomeren

1.5 g (8.2 mmol) α-Glucosylfluorid werden in 2 ml 0.05 M Natriumacetatpuffer gelöst (pH 6.5) immobilisierte α-Glucosidase (42 U, 73 % Immobilisierungsausbeute) hinzugegeben. Die Reaktionslösung wird dann bei 37°C unter Schütteln inkubiert. Der pH-Wert wird in einem Bereich zwischen 6 und 7 durch Zugabe von festem NaHCO₃ gehalten. Die Produktbildung kann durch DC (Propanol/Ethanol/Wasser, 5:3:2) gegen Standards (α-D-Glucosylfluorid, Glucose, Maltose, Maltotriose, Maltotetraose, Isomaltose) verfolgt werden. Nach 4 h wird die Reaktion abgebrochen und das immobilisierte Enzym durch Filtration entfernt, und die Lösung gefriergetrocknet.
Ausbeute: 1.41 g Rohprodukt
Das Reaktionsgemisch läßt sich mit einer RP18 HPLC-Säule (0.8 x 50 mm, 7 µm, Merck) mit Wasser als Eluationsmittel trennen.

| Retentionszeiten: | |
|---|---|
| Isomaltose (6 %) | 7.93 |
| Isomaltosylfluorid (25 %) | 7.12 |
| Panose (6 %) | 12.18 |

Isomaltosylfluorid: farbloser Sirup,
[α]_{D}²⁰ = +148.3 (c= 0.84 in Wasser)
¹H-NMR (D₂O): δ = 5.70 (dd, H-1), 4.96 (d, H-1');
J_{1,2} = 3.2, J_{1,F} = 52.5, J_{1',2'} = 4.0 Hz.

Das bisher nicht bekannte Isomaltosylfluorid wurde nach Acetylierung (Essigsäureanhydrid/Pyridin, 12 h, 20°C) auch als Heptaacetat charakterisiert.
6-O-(2,3,4,6-Tetra-O-acetyl-α-D-glucopyranosyl)-2,3,4-tri-O-acetyl-α-D-glucopyranosylfluorid: farbloser Sirup,
[α]_{D}²⁰ = +91.2 (c = 0.83 in Methylenchlorid)
¹H-NMR (CDCl₃): δ = 5.72 (dd, H-1), 4.90 (ddd, H-2), 5.47 (dd, H-3), 5.12 (dd, H-4), 4.12 (m, H-5), 3.74 (dd, H-6a), 3.61 (dd, H-6b), 5.15 (d, H-1'), 4.82 (dd, H-2'), 5.47 (dd, H-3'), 5.04 (dd, H-4'), 4.01 (m, H-5'), 4.04 (dd, H-6a'), 4.25 (dd, H-6b'), 1.89, 1.92, 1.94, 1.97, 2.01, 2.03, 2.12 ppm (7s, je 3H, OAc);
J_{1,2} = 3.7, J_{1,F} = 53.5, J_{2,F} = 25.1, J_{2,3} = 10.4, J_{3,4} = 10.0, J_{4,5} = 9.8, J_{5,6a} = 4.0, J_{5,6b} = 3.0, J_{6a,6b} = 11.6, J_{1',2'} = 3.5, J_{2',3'} = 10.5, J_{3',4'} = 9.0, J_{4',5'} = 9.7, J_{5',6a'} = 1.7, J_{5',6b'} = 4.5, J_{6a',6b'} = 12.0 Hz.

### Beispiel 2

### Umsetzung von α-D-Galactopyranosylfluorid

Die Immobilisierung der α-Galactosidase (α-D-Galactosidgalactohydrolase, E.C. 3.2.1.22) erfolgt wie in Beispiel 1 beschrieben.

500 mg (2.75 mmol) α-D-Galactosylfluorid werden in 1 ml 0.05 M Natriumacetatpuffer, pH 6.5 gelöst. Immobilisierte α-Galactosidase (6.5 U an 1 ml Träger, entsprechend Beispiel 1) wird hinzugefügt. Die Lösung wird unter Schütteln bei 37°C inkubiert. Der pH-Wert der Reaktionslösung wird durch Zugabe von Kationenaustauscher (Polystyrol-Sulfonsäureharz, z.B. ®Dowex 50Wx8) in einem Bereich zwischen pH 6 und 7 gehalten. Die Produktbildung kann durch Dünnschichtchromatographie detektiert werden (Propanol/Ethanol/Wasser, 5:3:2).

Nach 24 h ist keine neue Produktbildung mehr zu erkennen. Die Reaktion wird abgebrochen, indem das immobilisierte Enzym durch Filtration entfernt wird. Das Produktgemisch wird abschließend zur weiteren Aufarbeitung gefriergetrocknet.
Galactobiosylfluorid: farbloser Sirup
[α]_{D}²⁰ = + 82.3 (c= 0.74 in Wasser)
¹H-NMR (D₂O): δ = 5.68 (dd, H-1), 4.91 (d, H-1');
J_{1,2} = 2.7, J_{1,F} = 51.2, J_{1',2'} = 3.8 Hz.

Das Rohprodukt wird nach Acetylierung (5 ml Pyridin, 3 ml Essigsäureanhydrid, 12 h, 20°C) an einer Sephadex LH-20 Säule (2.5 x 60 cm) mit Essigester als Laufmittel getrennt.

Ausbeute 6-O-(2,3,4,6-Tetra-O-acetyl-α-D-galactopyranosyl)-2,3,4-tri-O-acetyl-α-D-galactopyranosylfluorid: 82 mg (9%) Sirup
[α]_{D}²⁰ = +101.4 (c = 0.84 in Chloroform)
¹H-NMR (CDCl₃): δ = 5.72 (dd, H-1), 5.11 (ddd, H-2), 5.03 (dd, H-3), 5.26 (dd, H-4), 3.93 (ddd, H-5), 3.71 (dd, H-6a), 3.39 (dd, H-6b), 4.91 (d, H-1'), 5.03 (dd, H-2'), 5.27 (dd, H-3'), 5.26 (dd, H-4'), 4.09 (ddd, H-5'), 4.15 (dd, H-6a'), 4.31 (dd, H-6b'), 1.87, 1.88, 1.96, 2.02, 2.05, 2.09, 2.12 ppm (7s, je 3H, OAc);
J_{1,2} = 2.7, J_{1,F} = 53.3, J_{2,F} = 24.4, J_{2,3} = 10.8, J_{3,4} = 2.8, J_{4,5} = 9.6, J_{5,6a} = 7.6, J_{5,6b} = 5.8, J_{6a,6b} = 9.1, J_{1',2'} = 3.5, J_{2',3'} = 9.8, J_{3',4'} = 2.8, J_{4',5'} = 9.8, J_{5',6a'} = 7.0, J_{5',6b'} = 8.8, J_{6a',6b'} = 9.0 Hz.

## Patentansprüche

1. Verfahren zur Herstellung von Disaccharidfluoriden, dadurch gekennzeichnet, daß α-Glycosylfluoride in Reaktionslösungen mit 30 bis 60 Gew.-% Substrat mit α-Glucosidase oder α-Galactosidase inkubiert werden.

2. Verfahren nach Anspruch 1, dadurch gekennzeichnet, daß in Reaktionslösungen mit 40 bis 50 Gew.-% Substrat inkubiert wird.

3. Verfahren nach Anspruch 1 oder 2, dadurch gekennzeichnet, daß die Reaktion bei 20 bis 60°C durchgeführt wird.

4. Verfahren nach Anspruch 3, dadurch gekennzeichnet, daß die Reaktion bei 30 bis 40°C durchgeführt wird.

5. Verfahren nach einem oder mehreren der Ansprüche 1 bis 4, dadurch gekennzeichnet, daß die Reaktion bei einem pH-Wert von 5 bis 8 durchgeführt wird.

6. Verfahren nach Anspruch 5, dadurch gekennzeichnet, daß die Reaktion bei einem pH-Wert von 6 bis 7 durchgeführt wird.

7. Verfahren nach einem oder mehreren der Ansprüche 1 bis 6, dadurch gekennzeichnet, daß α-D-Glucosylfluorid bzw. α-D-Galactosylfluorid als Substrat eingesetzt wird.

8. Die Verbindungen Isomaltosylfluorid der Formel I 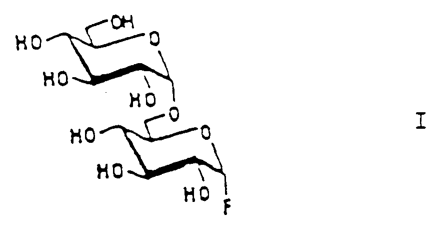 und Galactobiosylfluorid der Formel II

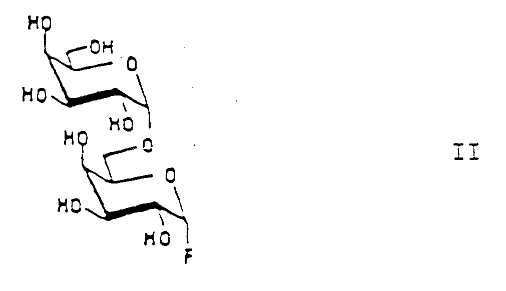

## Claims

1. A process for the preparation of disaccharide fluorides, which comprises incubation of α-glycosyl fluorides in reaction solutions containing 30 to 60% by weight of substrate with α-glucosidase or α-galactosidase.

2. The process as claimed in claim 1, wherein reaction solutions containing 40 to 50% by weight of substrate are incubated.

3. The process as claimed in claim 1 or 2, wherein the reaction is carried out at 20 to 60°C.

4. The process as claimed in claim 3, wherein the reaction is carried out at 30 to 40°C.

5. The process as claimed in one or more of claims 1 to 4, wherein the reaction is carried out at a pH of 5 to 8.

6. The process as claimed in claim 5, wherein the reaction is carried out at a pH of 6 to 7.

7. The process as claimed in one or more of claims 1 to 6, wherein the substrate used is α-D-glucosyl fluoride or α-D-galactosyl fluoride.

8. The compounds isomaltosyl fluoride of the formula I 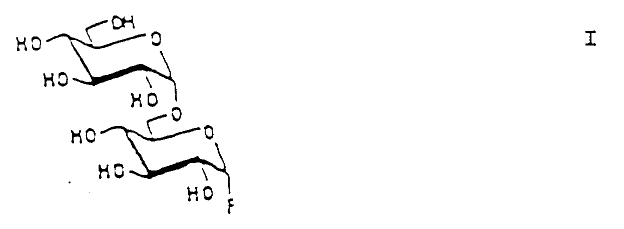 and galactobiosyl fluoride of the formula II

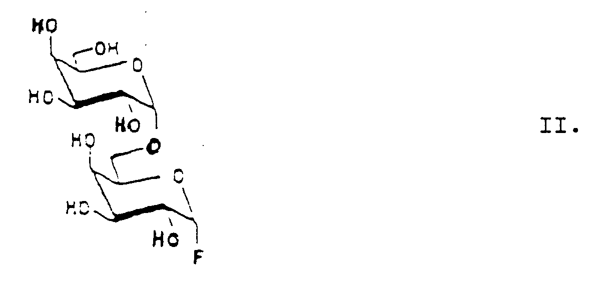

## Revendications

1. Procédé pour la préparation de fluorures de disaccharides, caractérisé en ce que l'on met à incuber des fluorures d'α-glycosyle avec de l'α-glucosidase ou de l'α-galactosidase dans des solutions réactionnelles contenant de 30 à 60 % en poids de substrat.

2. Procédé selon la revendication 1, caractérisé en ce que l'on met à incuber dans des solutions réactionnelles contenant de 40 à 50 % en poids de substrat.

3. Procédé selon la revendication 1 ou 2, caractérisé en ce que la réaction est effectuée à 20-60°C.

4. Procédé selon la revendication 3, caractérisé en ce que la réaction est effectuée à 30-40°C.

5. Procédé selon une ou plusieurs des revendications 1 à 4, caractérisé en ce que la réaction est effectuée à un pH de 5 à 8.

6. Procédé selon la revendication 5, caractérisé en ce que la réaction est effectuée à un pH de 6 à 7.

7. Procédé selon une ou plusieurs des revendications 1 à 6, caractérisé en ce que l'on utilise en tant que substrat du fluorure d'α-D-glucosyle ou du fluorure d'α-D-galactosyle.

8. Les composés fluorure d'isomaltosyle de formule I 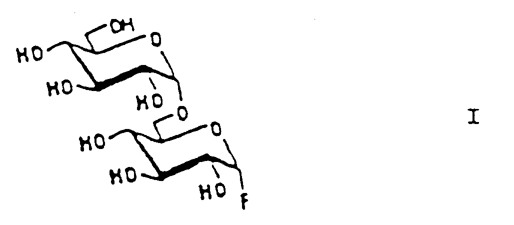 et fluorure de galactobiosyle de formule II
